# EUROPEAN PATENT APPLICATION

(11) **EP 3 970 753 A1**
(43) Date of publication of application: **23.03.2022**
(21) Application number: 20464012.2
(22) Date of filing: 09.10.2020
(51) Int. Cl.: A61K 47/69, A61K 9/51, A61K 47/00

(54) **MANGANESE-DOPPED NANOSTRUCTURED CARBON DOTS WITH APPLICATIONS IN ANTITUMORAL TREATMENTS AND MEDICAL IMAGING**

(30) Priority: 18.09.2020 RO 202000591
(71) Applicant: Universitatea Tehnica "Gheorghe Asachi" Din Iasi, 700050 Iasi (RO)
(72) Inventor: Stan, Corneliu-Sergiu, 700050 Iasi (RO); Secula, Marius-Sebastian, Judetul Iasi (RO)

(57) **Abstract**

The invention refers to Carbon Dots doped with Mn²⁺ having anti-tumor activity allowing at the same time the increase of contrast in medical MRI imaging, thus obtaining a theranostic platform for treatment and investigation; as well as a process for their preparation. These Carbon nanostructures are prepared through the partial pyrolysis of Mn(II)-N-hydroxyphthalimide complex under controlled thermal and exposure conditions. The resulting initial dispersion is centrifuged consecutively to obtain Mn²⁺ doped Carbon Dots nanostructures having dimensional distribution in the range of 10-60 nm. Then, they are freeze dried and re-dispersed in aqueous medium to the concentration required for the aimed applications.

## Description

The invention refers to Carbon Dots doped with Mn²⁺ having anti-tumor activity allowing at the same time the increase of contrast in medical MRI imaging, thus obtaining a theranostic platform for treatment and investigation; as well as a process for their preparation. These Carbon nanostructures are prepared by partial pyrolysis of a Mn²⁺ complex with N-hydroxyphthalimide. The anti-tumor effect of Carbon Dots obtained by partial pyrolysis of imide precursors (N-Hydroxysuccinimide, N-Hydroxyphthalimide) [1,2] has been previously demonstrated by means of *in vitro* and *in vivo* tests [3 -5]. The presence of Mn²⁺ in the specific chemical configuration of these carbon nanostructures leads to an improvement of the contrast in medical imaging recorded by NMR techniques.

There are already known Carbon Dots nanostructures doped with Fe³⁺, having intense photoluminescent emission in the blue and green area of the visible spectrum, as a function of the hydration degree, obtained by partial pyrolysis of a Fe³⁺-N-Hydroxyphthalimide complex [6]. Photoluminescent nanostructures of Carbon Dots doped with Mn²⁺ were prepared by a hydrothermal process from sodium citrate and manganese chloride with applications in the detection of iron ions [7]. Mn²⁺-doped Carbon Dots having intense photoluminescence in the green - orange are of the Visible spectrum, pH dependent, with potential applications in the fields of optical devices and pigments for fingerprinting, were prepared by a solvothermal process from pyromellitic acid and chloride. manganese [8]. In other similar approaches there were prepared Zn²⁺-doped Carbon Dots, having applications in bone therapy and medical imaging, [9] and Cu²⁺-doped Carbon Dots obtained by partial thermal degradation of citric acid in the presence of copper nitrate, applied for glucose detection from biological samples [10].

Carbon Dots nanostructures with anti-tumor action were prepared by hydrothermal processing of walnut oil [11]. The anti-tumor activity of Carbon Dots nanostructures obtained by pyrolytic processing of cellulose resulting from bamboo leaves subsequently loaded with doxorubicin and 4-carboxybenzylboronic acid was highlighted [12]. In a similar approach, doxorubicin-loaded Carbon Dots were prepared by thermal processing of sodium citrate and urea mixture [13]. Sulfur-doped Carbon Dots present interest for the non-invasive photodynamic therapy in tumors with oral localization, being prepared by the hydrothermal method using polythiophene as precursor [14]. Ru²⁺ doped Carbon Dots antitumor theranostic platforms were prepared through the hydrothermal method using citric acid and a 5-amino-1,10-phenanthroline ruthenium (II) complex as precursors [15].

The contrast agents currently used in MRI medical imaging are mostly chelated chemical complexes of some elements with paramagnetic properties such as Gd³⁺ or Mn²⁺ [16,17].

The main disadvantages of Carbon Dots nanostructures applied as anti-tumor theranostic platforms reported so far consist in:
- their anti-tumor activity is relatively limited or obtained by the addition of classical anti-tumor compounds (e.g. doxorubicin).
- their use in associated medical imaging is limited to fluorescence techniques, and cannot be used in MRI investigation techniques.
- in some cases there is an increased toxicological potential due to the additional content of organic or organo-metallic compounds [18].

The most similar Carbon Dots nanostructures containing cations from the transition groups are those doped with Fe³⁺ obtained from a Fe³⁺-N-Hydroxyphthalimide complex at a metal / ligand combination ratio of 1/3 [6] and those prepared from citric acid and a complex Ru (II) - 5-amino-1,10-phenanthroline [15].

The technical problem that this invention aims to solve is to obtain Carbon Dots that present simultaneously a relevant anti-tumor effect and the possibility of using them as a contrast agent in medical investigations by NMR techniques while maintaining a low level of toxicity.

The solution to the technical problem is to obtain Carbon Dots doped with Mn²⁺ by pyrolytic processing under controlled conditions of thermal exposure of an Mn²⁺ complex with N-Hydroxyphthalimide at a metal / ligand combination ratio of 1/2, followed by the initial dispersion in aqueous medium, dimensional selection, drying and redispersion in a medium compatible with their use in medical applications as an anti-tumor and contrast agent in MRI imaging.

The main advantages of the proposed invention are:
- obtaining a theranostic platform for medical investigation by MRI imaging and effective anti-tumor treatment.
- preliminary investigations indicate a low degree of toxicity; the anti-tumor therapeutic action minimally affects healthy cells and tissues.
- simple and scalable preparation process for industrial production capacities.

According to the invention, the preparation of anti-tumor theranostic nanostructures involves partial pyrolysis at a temperature of 235-240 °C, in an N₂ atmosphere of a coordination complex (purified and dried) obtained in a medium consisting of a water / ethanol mixture through the reaction between MnCl₂ and N-hydroxyphthalimide at a combination ratio of 1/2. The complex is prepared by dissolving in a water / ethanol mixture (60/40% by volume) under stirring conditions the appropriate amounts of MnCl₂ and N-Hydroxyphthalimide to obtain a combination ratio of 1/2. After completion of the complexation reaction, the resulting precipitate is washed 2-3 times with distilled water and dried under vacuum. The Mn(II)-N-Hydroxyphthalimide complex is thermally processed by a process of partial pyrolysis in a protective atmosphere of N₂ at a temperature of 235-240 °C. The thermal exposure time of (Mn(II-N-Hydroxyphthalimide) precursor is 6 min (+/- 30s), and is essential to obtain Carbon Dots with physical-chemical configuration favorable for application as MRI contrast agent and anti-tumor treatment. After completion of the pyrolysis phase, the resulting product is suddenly flooded with distilled water at 5-6 °C, the resulting dispersion being collected from the pyrolysis vessel.

The aqueous dispersion is subjected to successive centrifugation operations for dimensional selection, the clear-looking supernatant collected containing Mn²⁺ doped Carbon Dots with a dimensional distribution in the range of 10-60 nm. The resulting aqueous dispersion is frozen at - 23 ÷ -18 °C and then freeze dried, resulting dried Mn²⁺ doped Carbon Dots in the form of a fine powder with a yellow-brown appearance. For administration, the resulting powder is re-dispersed in water at the concentration necessary to obtain the therapeutic effect or according to the specific requirements of MRI investigation.

The following is an embodiment of the invention for obtaining Mn²⁺ doped Carbon Dots with anti-tumor activity and use as a contrast agent in medical NMR imaging. The typical experimental procedure involves the prior preparation of the Mn (II)-N-Hydroxyphthalimide complex by dissolving under vigorous stirring in a water / ethanol mixture (30 ml / 20 ml) 0.5 g of anhydrous MnCl₂ and 1.3 g of N-Hydroxyphthalimide. The complexation reaction proceeds at 40-45°C under moderate stirring over a period of 20-24 h, resulting a white-yellow precipitate. The collected precipitate is washed with double-distilled water in three successive stages and then dried under vacuum. The entire amount of the resulted Mn(II)-N-Hydroxyphthalimide complex in the form of a dry powder is thermally processed in a quartz container provided with a heating jacket. The pyrolytic process takes place in a protective atmosphere of N₂ at a temperature of 235-240 °C for a period of 6 min (+/- 30s). After completion of the heat exposure stage, the resulting product in the quartz container is suddenly flooded with approx. 100 ml of double-distilled water at a temperature of 5-6 °C. The raw aqueous dispersion thus obtained is discharged from the quartz vessel and ultrasonically processed for 10 min. The aqueous dispersion is further centrifuged at a speed of 10,000 RPM for 10 min. The supernatant collected after the first centrifugation is again centrifuged at 15000 rpm. for 15 min. The supernatant with a clear appearance obtained after the final centrifugation contains Mn²⁺ doped Carbon Dots with a dimensional distribution in the range of 10-60 nm. Next, the aqueous dispersion is frozen at - 23 ÷ -18 °C and freeze dried. Mn²⁺ doped Carbon Dots in the aqueous dispersion have a tendency to agglomerate, therefore, the cooling operation for freezing must be initiated immediately after the last centrifugation operation. After lyophilization, Mn²⁺ doped Carbon Dots are obtained in the dry state in the form of a fine powder with yellow-brown appearance. The resulting amount of approx. 350 mg of dry powder is stored in sealed recipes and re-dispersed in aqueous medium at the required concentration.

### References

[1] C. S Stan, P. Horlescu, L. E. Ursu, M. Popa, C. Albu, Facile preparation of highly luminescent composites by polymer embedding of carbon dots derived from N-hydroxyphthalimide, Springer- Journal of Material Science 52(1), pp. 185-196, 2017. doi 10.1007/s10853-016-0320-y
[2] C. S. Stan, A. Coroaba, M. Popa, C. Albu, D. Sutiman, One step synthesis of fluorescent Carbon Dots through pyrolysis of N-hydroxysuccinimide, RSC-Journal of Materials Chemistry C 3, pp.789-795, 2014, doi: 10.1039/C4TC02382J
[3] C. E. Tiron, G. Luta, M. Butura, F. Zugun-Eloae, C. S. Stan, A. Coroaba, E. L. Ursu, G. D. Stanciu, A. Tiron, NHF-derived carbon dots: prevalidation approach in breast cancer treatment, Nature-Scientific Reports 10:12662, 2020, doi.10.1038/s41598-020-69670-z
[4] C. L. Savin, C. Tiron, E. Carasevici, C. S. Stan, S. A. Ibanescu, B. Simionescu, C. A. Peptu, Entrapment of N-Hydroxyphthalimide Carbon Dots in Different Topical Gel Formulations: New Composites with Anticancer Activity, Pharmaceutics 11(7):303, 2019. doi: 10.3390/pharmaceutics11070303
[5] C. E. Tiron, F. Zugun-Eloae, C. A. Peptu, C. S. Stan, A. Tiron, Imide derived carbon dots-a new promissing approach in cancer treatment, Nano Sci Nano Tech Ind. J., 2019
[6] C. S. Stan, A. Coroaba, E. L. Ursu, M. S. Secula, B. C. Simionescu, Fe(III) doped carbon nanodots with intense green photoluminescence and dispersion medium dependent emission, Nature-Scientific Reports 9, 18893, 2019 doi: 10.1038/s41598-019-55264-x
[7] Sun, S. et al. Highly luminescence manganese doped carbon dots. Chin. Chem. Lett. 30, 1051-1054, 2019, doi.10.1016/j.cclet.2019.01.014
[8] R. Kumari, K. Pal, P. Karmakar, S Kumar Sahu, pH-Responsive Mn-Doped Carbon Dots for White-Light-Emitting Diodes, Fingerprinting, and Bioimaging, ACS Appl. Nano Mater. (2)9, 5900-5909, 2019, doi.10.1021/acsanm.9b01335
[9] Y. Meng, M. Yang, X. Liu, W. Yu, B. Yang, Zn2+-Doped Carbon Dots, a Good Biocompatibility Nanomaterial Applied for Bio-Imaging and Inducing Osteoblastic Differentiation in vitro, Nano 14(03), 1950029, 2019, doi.10.1142/S1793292019500292
[10] Y. Duan, Y. Huang, S. Chen, W. Zuo, B. Shi, Cu-Doped Carbon Dots as Catalysts for the Chemiluminescence Detection of Glucose, ACS Omega 4(6), 9911-9917, 2019, doi.10.1021/acsomega.9b00738
[11] E. Arkan, A. Barati, M. Rahmanpanah, L. Hosseinzadeh, S. Moradi, M. Hajialyani, Green Synthesis of Carbon Dots Derived from Walnut Oil and an Investigation of Their Cytotoxic and Apoptogenic Activities toward Cancer Cells, Adv. Pharm. Bull. 8(1), pp.149-155, 2018, doi: 10.15171/apb.2018.018
[12] M. Z. Fahmi, A. Haris, A. J. Permana, D. L. Nor Wibowo, B. Purwanto, Y. L. Nikmah, A. Idris, Bamboo leaf-based carbon dots for efficient tumor imaging and therapy, RSC Adv., 8, pp. 38376-38383, 2018, doi.10.1039/C8RA07944G
[13] Y. Sun, S. Zheng, L.Liu, Y. Kong, A. Zhang, K. Xu, C. Han, The Cost-Effective Preparation of Green Fluorescent Carbon Dots for Bioimaging and Enhanced Intracellular Drug Delivery, Nanoscale Res. Lett., 15, 55, 2020, doi.10.1186/s11671-020-3288-0
[14] Q. Li, R. Zhou, Y. Xie, Y. Li, Y. Chen, X. Cai, Sulphur-doped carbon dots as a highly efficient nano-photodynamic agent against oral squamous cell carcinoma, Cell Proliferation 53(4), 2020, doi.10.1111/cpr.12786
[15] L. Yue, H. Li, Q. Sun, J. Zhang, X. Luo, F. Wu, X. Zhu, Red-Emissive Ruthenium-Containing Carbon Dots for Bioimaging and Photodynamic Cancer Therapy, ACS Appl. Nano Mater. 3(1), pp. 869-876, 2020, doi.10.1021/acsanm.9b02394
[16] Y-D Xiao, R. Paudel, J. Liu, C. Ma, Z-S. Zhang, S-K. Zhou, MRI contrast agents: Classification and application (Review), International J. of Molecular Medicine 38(5), pp. 1319-1326, 2016, doi: 10.3892/ijmm.2016.2744
[17] H-K Kim, G. H. Lee, Y. Chang, Gadolinium as an MRI contrast agent, Future Medicinal Chem. 10(6), 2018, doi.10.4155/fmc-2017-0215
[18] D. V. Bower, J. Richter, K. Johannes, H. von Tengg-Kobligk, J. Heverhagen, M. V. Runge, Gadolinium-Based MRI Contrast Agents Induce Mitochondrial Toxicity and Cell Death in Human Neurons, Toxicity Increases With Reduced Kinetic Stability of the Agent, Investigative Radiology 54(8), pp. 453-463, 2019 doi: 10.1097/RLI.0000000000000567

## Claims

1. Manganese-doped Carbon Dots usable in antitumor treatments and MRI medical imaging, **characterized by:** they are obtained by the thermal processing of Mn(II)-N-hydroxyphthalimide complex.

2. The process of obtaining manganese doped Carbon Dots usable in antitumor treatments and MRI medical imaging, **characterized by:** it involves partial pyrolysis at 235-240°C, within 6 min duration of thermal exposure, of a Mn(II)-N-Hydroxyphthalimide complex followed by flooding the resulted primary dispersion with distilled water cooled down to 5-6°C, centrifugation of the dispersion obtained for the dimensional selection of nanostructures within 10-60 nm range, freeze-drying to obtain them in dry state powder and further re-dispersion in biologically compatible media.

## Amended claims

### Amended claims in accordance with Rule 137(2) EPC.

1. Manganese-doped Carbon Dots derived from of Mn(II) complex with N-Hydroxyphthalimide suitable for anti-tumor treatments and MRI medical imaging, **characterized by:** their particular structural configuration achieved through the herein described thermal processing of the mentioned complex.

2. The process of obtaining manganese doped Carbon Dots derived from of a Mn(II) complex with N-Hydroxyphthalimide suitable for anti-tumor treatments and MRI medical imaging, **characterized by:** partial pyrolysis at 235-240°C, within 6 min duration of thermal exposure, of the mentioned complex followed by rapid flooding with distilled water cooled down to 5-6°C and further centrifugation of the dispersion for the dimensional selection followed by freeze-drying and re-dispersion of the dried product in biologically compatible media.
